# EUROPEAN PATENT SPECIFICATION

(11) **EP 3 943 035 B1**
(45) Date of publication and mention of the grant of the patent: **11.09.2024**
(21) Application number: 20910459.5
(22) Date of filing: 26.10.2020
(51) Int. Cl.: B32B 5/02, A61B 46/23, A61M 25/00, A61B 46/00, B32B 7/09, B32B 5/26, B32B 7/12, B32B 27/32, B32B 27/12, A61M 25/09

(54) **ENDOVASCULAR DRAPES WITH INTEGRATED WATERPROOF GUIDEWIRE DISPENSER**
ENDOVASKULÄRE ABDECKTÜCHER MIT INTEGRIERTEM WASSERDICHTEM FÜHRUNGSDRAHTSPENDER
DRAPS ENDOVASCULAIRES AVEC DISTRIBUTEUR DE FIL-GUIDE ÉTANCHE À L'EAU INTÉGRÉ

(30) Priority: 30.12.2019 CN 201911387548
(43) Date of publication of application: 26.01.2022
(73) Proprietor: Cobes Health Care (Hefei) Co., Ltd., Feidong Hefei, Anhui 231600 (CN)
(72) Inventor: GAO, Ming, Hefei, Anhui 231600 (CN); PATRICK, Lestoquoy, Attiches 59551 (FR)
(74) Representative: Kluin Patent
(86) International application number: PCT/CN2020/123548
(87) International publication number: WO 2021/135556

(56) References cited:
- WO-A1-2011/025439
- WO-A1-2013/036387
- WO-A1-99/33408
- CN-A- 110 946 656
- CN-B- 103 796 610
- CN-U- 206 630 674
- CN-U- 208 892 770
- US-A- 5 358 495
- US-A1- 2017 361 067
- US-A1- 2019 275 304
- US-B1- 6 436 085

## Description

### Technical field

The present invention relates to the technical field of guide wires, and in particular to a drape including an integrated guide wire use device.

### Technical background

At present, guide wires in endovascular surgery are very important and an important part of surgery. These guide wires have broad application prospects in angiography, coronary angiography, valve replacement, coronary artery replacement and so on. For all these steps, one or two or more guide wires are used now. They are very expensive and difficult to protect. Using them in the market's existing technology can solve different problems. First, it is the length issue of the guide wire and its toughness problem. According to the professional requirements, the length may be between 20 to 150 cm, and they are used in a PE pipe.

The existing urinary catheter is pulled out and inserted into a large tray or a special tray including physiological saline. The use of this guide wire basin is risky. Due to the length and its flexibility, if the operation is not careful, the guide wire will jump out of the guide wire basin, thereby leading to product contamination. These specific guide wire basins require 10 to 15 cm in height. The packaging for special purposes is a very large volume, which increases the cost of transportation, sterilization, storage and physiological saline used in the clinical process. If the guide wire needs to be replaced, it must always be placed in the guide wire basin, which is very inconvenient to use.

US 5358495 A discloses a guidewire insertion assembly.

### Summary of the invention

### (I) Technical Problem Solved

In view of the shortcomings of the prior art, the present invention provides a drape including an integrated guide wire use device, which solves the following problem: the packaging for special purposes is a very large volume, which increases the cost of transportation, sterilization, storage and physiological saline used in the clinical process, and if the guide wire needs to be replaced, it must always be placed in the guide wire basin, which is very inconvenient to use.

### (II) Technical Solution

In order to achieve the above objective, the present invention is implemented as in claim 1.

Preferably, the drape body comprises a surface layer, a water-absorbing layer, an antibacterial layer and a bottom lining layer, a bottom of the surface layer is stitched and connected to a top of the water-absorbing layer.

Preferably, a bottom of the water-absorbing layer is stitched and connected to a top of the antibacterial layer, and a bottom of the antibacterial layer is stitched and connected to a top of the bottom lining layer.

Preferably, the water-absorbing layer is woven by blending polyacrylonitrile fiber and polyethylene oxide fiber, the antibacterial layer uses bamboo charcoal fiber as a raw material, and the bottom lining layer uses pure cotton as a raw material.

Preferably, the surface of the drape body is stitched and connected with a fixing line.

Preferably, the guide wire bag uses PE as a raw material.

Preferably, the surface layer comprises a first surface layer, a second surface layer and a third surface layer, the first surface layer is separately stitched and connected to surfaces of the second surface layer and the third surface layer, and the second surface layer is stitched and connected to the surface of the third surface layer.

Preferably, the first surface layer uses cobes21G PP+70G airlaid as a raw material, the second surface layer uses Transparent PE 0.05 mm as a raw material, and the third surface layer uses cobes2730/pe27a+spu30B as a raw material.

The drape body is an existing one, which is an infection control non-woven fabric covered with an adhesive used in an operating room, and is generally made of a non-woven fabric or a PE composite non-woven fabric. The present invention improves the drape body by adding a water-absorbing layer, an antibacterial layer and a bottom lining layer, and setting the surface layer of the drape body as a three-layer layered structure comprising a first surface layer and a second surface layer and the third surface layer. In general, the first surface layer is a composite layer compounded by PP (polypropylene) and airlaid (dust-free paper) through an adhesive, a transparent polyethylene layer is used as the second surface layer, and the third surface layer is a composite layer compounded by PP (polypropylene) and polyethylene (PE) through an adhesive.

The above-mentioned PP, PE, and dust-free paper are all commercially available existing products.

In the present application, in particular, the first surface layer uses cobes21G PP+70G airlaid as a raw material, that is, a composite layer compounded by PP (polypropylene) with a gram weight of 21 grams per square meter and airlaid (dust-free paper) with a gram weight of 70 grams per square meter through an adhesive produced by the applicant is used; the second surface layer uses Transparent PE 0.05mm as a raw material, which refers to transparent PE with a thickness of 0.05mm made by a blow molding method; and the third surface layer uses cobes2730/pe27a+spu30B as a raw material, which refers to a composite layer compounded by PE (polyethylene) with a gram weight of 27 grams per square meter and blue PP (polypropylene) with a gram weight of 30 grams per square meter through an adhesive produced by the applicant using a casting method (code: cobes2730).

In particular, the blow molding method is to extrude plastic into a thin tube, then blow the plastic to a required thickness by high-pressure air while it is hot, and then cool it to shape and rewind.

The casting method is to melt PE particles through a screw, flow out at a required width through a mold head, perform molding by a calender roll, and cool it to shape, so that a finished product of cast film is finished.

PP is a spunbonded nonwoven fabric. After a PP polymer is extruded and stretched to form continuous filaments, the continuous filaments are then laid into a web, and then it is subjected to a reinforcement treatment of thermal bonding or chemical bonding, so that the web turns into a non-woven fabric.

Airlaid is also called an Airlaid pulp nonwoven fabric, which is one of dry nonwoven fabrics. Wood pulp fiber is pulverized and stirred, air-laid into a web and then molded, bonded by a chemical adhesive, and then baked and consolidated into a fabric.

Preferably, an outer side of the protective bag is fixedly provided with a convex block, and a material of the convex block is polyethylene.

Preferably, a right end part of the guide wire bag is a horn-shaped connector that expands toward the end part, and the disinfection connection port and the drain plug are provided on the horn-shaped connector, and a protective film is encapsulated at a port of the disinfection connection port.

The protective film is made of a microporous, breathable and waterproof material, such as a breathable PE film, or a composite film of breathable PP and breathable PE. It can isolate large water molecules and permeate small gas molecules. When sterilizing, a sterilant gas can enter the guide wire bag through the protective film for sterilization, but it is difficult for other water molecules, bacteria and other macromolecules to enter, thereby playing the role of barrier and protection.

### (III) Beneficial Effects

The present invention provides a drape including an integrated guide wire use device. Compared with the prior art, the present invention has the following beneficial effects:
(1) In the angiography sheet with the integrated guide wire bag, the back of the guide wire bag is movably connected to the surface of the drape body; the inner cavity of the guide wire bag is fixedly connected with the guide wire insertion block; the guide wire insertion block uses the sponge or soft rubberized fabric as the raw material; the left side of the guide wire bag is movably connected with the protective bag; the surface of the protective bag is adhered with the thermoplastic rubber folding film, and the surface of the thermoplastic rubber folding film is adhered to the surface of the guide wire bag; and the surface of the protective bag is glued and connected with the adhesive tape. As a result, the drape body with the guide wire bag can protect the guide wire during use. There is no risk of falling during use, and there is no need to use multiple guide wires. Moreover, the guide wire bag can allow single or multiple guide wires to be inserted into one or more guide wire bags, which is very simple and convenient to use, and reduces the consumption of physiological saline.
(2) In the angiography sheet with the integrated guide wire bag, the surface of the guide wire bag is provided with the disinfection connection port, the right side of the guide wire bag is sealed and connected with the drain plug, the back of the guide wire bag is glued and connected with the adhesive fabric, and the back of the adhesive fabric is adhered to the surface of the drape body. As a result, there is no need to use a guide wire basin, and at the same time, the costs of disinfection, storage and transportation of the radiography bag are reduced. The size of the guide wire bag can be adjusted according to the size and width of the guide wire and it can be applied used in various drapes. It is very convenient to use and suitable for a variety of working environments.
(3) In the angiography sheet with the integrated guide wire bag, the drape body comprises the surface layer, the water-absorbing layer, the antibacterial layer and the bottom lining layer; the bottom of the surface layer is stitched and connected to the top of the water-absorbing layer; the bottom of the water-absorbing layer is stitched and connected to the top of the antibacterial layer; and the bottom of the antibacterial layer is stitched and connected to the top of the bottom lining layer; the water-absorbing layer is woven by blending polyacrylonitrile fiber and polyethylene oxide fiber; the antibacterial layer uses bamboo charcoal fiber as the raw material, and the bottom lining layer uses pure cotton as the raw material. As a result, the water and stains on the drape body are absorbed through the water-absorbing layer, the stains and water are blocked by the water-absorbing layer, and the growth of bacteria in the drape body can be prevented through the sterilization effect of the antibacterial layer.

### Brief description of the drawings

Fig. 1 is a top view of the structure according to the present invention;
Fig. 2 is a top view of the structure of the guide wire bag according to the present invention;
Fig. 3 is a bottom view of the structure of the guide wire bag according to the present invention;
Fig. 4 is a side view of the structure of the guide wire bag according to the present invention;
Fig. 5 is a front view of the structure of the drape body according to the present invention; and
Fig. 6 is a top view of a second structure of the guide wire bag structure according to the present invention.

In the figures, 1-drape body, 2-guide wire bag, 3-guide wire insertion block, 4-protective bag, 5-thermoplastic rubber folding film, 6-adhesive tape, 7-drain plug, 8-adhesive fabric, 9-fixing line, 10-disinfection connection port, 11-surface layer, 12-water-absorbing layer, 13-antibacterial layer, 14-bottom lining layer, 111-first surface layer, 112-second surface layer, 113-third surface layer, 15-convex block.

### Detailed description of the embodiments

The technical solutions in the embodiments of the present invention will be clearly and completely described below with reference to the drawings in the embodiments of the present invention. Obviously, the described embodiments are only a part of the embodiments of the present invention, and not all the embodiments. All other embodiments obtained by an ordinary person skilled in the art on the basis of the embodiments of the present invention without creative efforts are within the scope of the present invention.

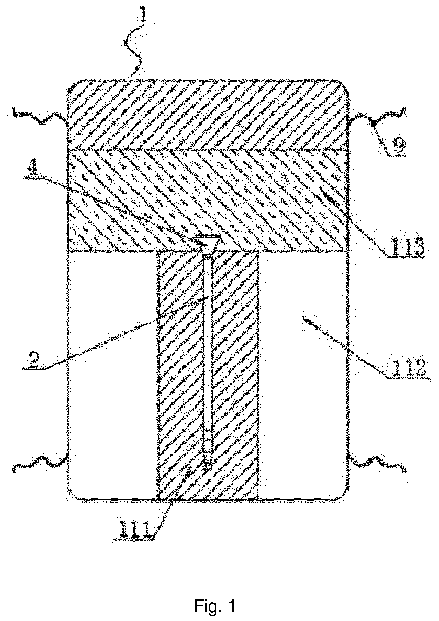

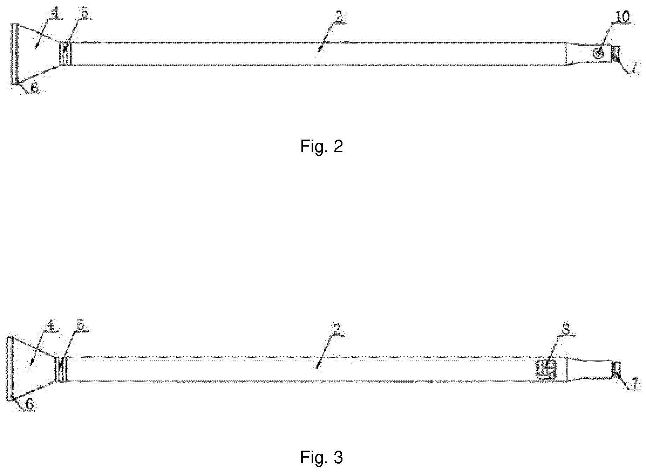

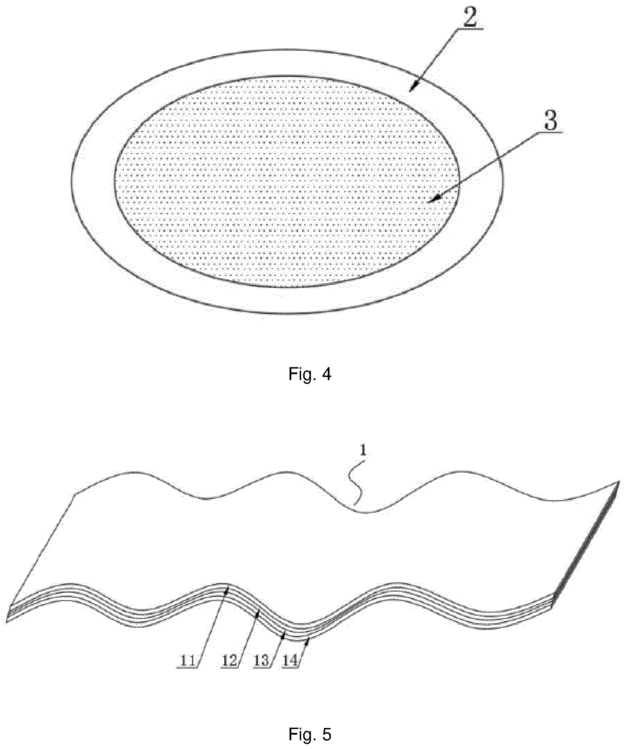

Referring to Figs. 1-5, an embodiment of the present invention provides a technical solution: a drape including an integrated guide wire use device, comprising a drape body 1 and a guide wire bag 2. The drape body 1 with the guide wire bag 2 can protect a guide wire during use. There is no risk of falling during use, and there is no need to use multiple guide wires. Moreover, the guide wire bag 2 can allow single or multiple guide wires to be inserted into one or more guide wire bags 2, which is very simple and convenient to use, and reduces the consumption of physiological saline. The guide wire bag 2 uses PE as a raw material. A surface of the drape body 1 is stitched and connected with a fixing line 9. The drape body 1 comprises a surface layer 11, a water-absorbing layer 12, an antibacterial layer 13 and a bottom lining layer 14. The surface layer 11 comprises a first surface layer 111, a second surface layer 112 and a third surface layer 113. The first surface layer 111 is separately stitched and connected to surfaces of the second surface layer 112 and the third surface layer 113, and the second surface layer 112 is stitched and connected to the surface of the third surface layer 113. The first surface layer 111 uses cobes21G PP+70G airlaid as a raw material, the second surface layer 112 uses Transparent PE 0.05 mm as a raw material, and the third surface layer 113 uses cobes2730/pe27a+spu30B as a raw material. A bottom of the water-absorbing layer 12 is stitched and connected to a top of the antibacterial layer 13. The water-absorbing layer 12 is woven by blending polyacrylonitrile fiber and polyethylene oxide fiber. The antibacterial layer 13 uses bamboo charcoal fiber as a raw material. The bottom lining layer 14 uses pure cotton as a raw material, and a bottom of the antibacterial layer 13 is stitched and connected to a top of the bottom lining layer 14. The bottom of the surface layer 11 is stitched and connected to a top of the water-absorbing layer 12. The water and stains on the drape body 1 are absorbed through the water-absorbing layer 12, the stains and water are blocked by the water-absorbing layer 12, and the growth of bacteria in the drape body 1 can be prevented through the sterilization effect of the antibacterial layer 13. The back of the guide wire bag 2 is movably connected to the surface of the drape body 1. An inner cavity of the guide wire bag 2 is fixedly connected with a guide wire insertion block 3. After a guide wire is inserted, the guide wire insertion block 3 will not be damaged. The guide wire insertion block 3 uses sponge or soft rubberized fabric as a raw material. A left side of the guide wire bag 2 is movably connected with a protective bag 4. A surface of the protective bag 4 is adhered with a thermoplastic rubber folding film 5, and a surface of the thermoplastic rubber folding film 5 is adhered to a surface of the guide wire bag 2. A surface of the protective bag 4 is glued and connected with an adhesive tape 6. The surface of the guide wire bag 2 is provided with a disinfection connection port 10. The disinfection of the bag is facilitated through the disinfection connection port 10. A right side of the guide wire bag 2 is sealed and connected with a drain plug 7, and the back of the guide wire bag 2 is glued and connected with an adhesive fabric 8. A back of the adhesive fabric 8 is adhered to the surface of the drape body 1. There is no need to use a guide wire basin, and at the same time, the costs of disinfection, storage and transportation of the radiography bag are reduced. The size of the guide wire bag can be adjusted according to the size and width of the guide wire, and it can be applied used in various drapes. It is very convenient to use and suitable for a variety of working environments. The technology of the present invention is to propose a drape with an integrated guide wire use device. The drape will include one or more guide wire bags 2 filled with physiological saline. In order to empty the physiological saline at the end of the operation, the physiological saline can be quickly discharged from the surface of the guide wire bag 2 through the drain plug 7. The guide wire bag 2 can replace the guide wire basin used in the prior art, and is fixed on the drape by means of gluing, and placed on the drape to avoid the crease area of the drape.

The first surface layer 111 mentioned above uses cobes21G PP+70G airlaid as a raw material, that is, a composite layer compounded by PP (polypropylene) with a gram weight of 21 grams per square meter and airlaid (dust-free paper) with a gram weight of 70 grams per square meter through an adhesive produced by the applicant is used. The second surface layer 112 uses Transparent PE 0.05mm as a raw material, which is made of transparent polyethylene with a thickness of 0.05mm. The third surface layer 113 uses cobes2730/pe27a+spu30B as a raw material, which refers to a composite layer compounded by PE (polyethylene) with a gram weight of 27 grams per square meter and blue PP (polypropylene) with a gram weight of 30 grams per square meter through an adhesive produced by the applicant using a casting method (code: cobes2730).

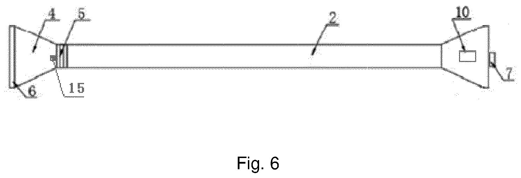

As shown in Fig. 6, a drape including an integrated guide wire use device comprises a drape body 1 and a guide wire bag 2. The drape body 1 with the guide wire bag 2 can protect a guide wire during use. There is no risk of falling during use, and there is no need to use multiple guide wires. Moreover, the guide wire bag 2 can allow single or multiple guide wires to be inserted into one or more guide wire bags 2, which is very simple and convenient to use, and reduces the consumption of physiological saline. The guide wire bag 2 uses PE as a raw material. A surface of the drape body 1 is stitched and connected with a fixing line 9. The drape body 1 comprises a surface layer 11, a water-absorbing layer 12, an antibacterial layer 13 and a bottom lining layer 14. The surface layer 11 comprises a first surface layer 111, a second surface layer 112 and a third surface layer 113. The first surface layer 111 is separately stitched and connected to surfaces of the second surface layer 112 and the third surface layer 113, and the second surface layer 112 is stitched and connected to the surface of the third surface layer 113. A composite layer compounded by PP (polypropylene) and airlaid (dust-free paper) through an adhesive is used as the first surface layer 111. A transparent polyethylene layer is used as the second surface layer 112. A composite layer compounded by PE (polyethylene) and blue PP (polypropylene) through an adhesive is used as the third surface layer 113. A bottom of the water-absorbing layer 12 is stitched and connected to a top of the antibacterial layer 13. The water-absorbing layer 12 is woven by blending polyacrylonitrile fiber and polyethylene oxide fiber. The antibacterial layer 13 uses bamboo charcoal fiber as a raw material. The bottom lining layer 14 uses pure cotton as a raw material, and a bottom of the antibacterial layer 13 is stitched and connected to a top of the bottom lining layer 14. The bottom of the surface layer 11 is stitched and connected to a top of the water-absorbing layer 12. The water and stains on the drape body 1 are absorbed through the water-absorbing layer 12, the stains and water are blocked by the water-absorbing layer 12, and the growth of bacteria in the drape body 1 can be prevented through the sterilization effect of the antibacterial layer 13. The back of the guide wire bag 2 is movably connected to the surface of the drape body 1. An inner cavity of the guide wire bag 2 is fixedly connected with a guide wire insertion block 3. After a guide wire is inserted, the guide wire insertion block 3 will not be damaged. The guide wire insertion block 3 uses sponge or soft rubberized fabric as a raw material. A left side of the guide wire bag 2 is movably connected with a protective bag 4. A surface of the protective bag 4 is adhered with a thermoplastic rubber folding film 5, and a surface of the thermoplastic rubber folding film 5 is adhered to a surface of the guide wire bag 2. An outer side of the protective bag 4 is fixedly provided with a convex block 15 for opening the thermoplastic rubber folding film 5. A material of the convex block 15 is polyethylene, which is fixed on the protective bag 4 by heat bonding. When in use, the guide wire is held with one hand, and the convex block 15 is held with the other hand to open the thermoplastic rubber folding film 5 and the guide wire insertion block 3 which are folded together, to facilitate the insertion of the guide wire. The surface of the protective bag 4 is glued and connected with an adhesive tape 6 so that it is bonded to the drape body. A right end part of the guide wire bag 2 is a horn-shaped connector that expands toward the end part, and the disinfection connection port 10 and the drain plug 7 are provided on the horn-shaped connector, and a protective film is encapsulated at a port of the disinfection connection port 10. The protective film is made of a breathable PE film. It can isolate large water molecules and permeate small gas molecules. When sterilizing, a sterilant gas can enter the guide wire bag through the protective film for sterilization, but it is difficult for other water molecules, bacteria and other macromolecules to enter, thereby playing the role of barrier and protection. Moreover, the back of the guide wire bag 2 is glued and connected with an adhesive fabric 8, and a back of the adhesive fabric 8 is adhered to the surface of the drape body 1. There is no need to use a guide wire basin, and at the same time, the costs of disinfection, storage and transportation of the radiography bag are reduced. The size of the guide wire bag can be adjusted according to the size and width of the guide wire, and it can be applied used in various drapes. It is very convenient to use and suitable for a variety of working environments. The technology of the present invention is to propose a drape with an integrated guide wire use device. The drape will include one or more guide wire bags 2 filled with physiological saline. In order to empty the physiological saline at the end of the operation, the physiological saline can be quickly discharged from the surface of the guide wire bag 2 through the drain plug 7. The guide wire bag 2 can replace the guide wire basin used in the prior art, and is fixed on the drape by means of gluing, and placed on the drape to avoid the crease area of the drape.

It needs to be noted that the relationships such as first and second herein are used merely to distinguish one entity or operation from another entity or operation, and do not necessarily require or imply that there are any such actual relationships or orders between these entities or operations. Furthermore, the terms "comprise," "comprising," "include" or "including" or any other variants are intended to include a non-exclusive inclusion, so that a process, a method, an article, or a device including a series of elements not only includes those elements but also includes other elements not explicitly listed, or further includes elements that are inherent to the process, the method, the article, or the device.

Although the embodiments of the present invention have been shown and described, it can be understood by those of ordinary skill in the art that various changes, modifications, substitutions and variations can be made to these embodiments without departing from the scope of the present invention. The scope of the present invention is defined by the appended claims.

## Claims

1. A drape including an integrated guide wire use device, comprising a drape body (1) and a guide wire bag (2), wherein a back surface of the guide wire bag (2) is movably connected to the drape body (1) wherein the back surface of the guide wire bag (2) is glued and connected with an adhesive fabric (8); and the adhesive fabric (8) is adhered to the drape body (1), and wherein an inner cavity of the guide wire bag (2) is fixedly connected with a guide wire insertion block (3); wherein the guide wire insertion block (3) has a material of sponge or soft rubberized fabric; and a first side of the guide wire bag (2) is movably connected with a protective bag (4); wherein the protective bag (4) is adhered with a thermoplastic rubber folding film (5), and the thermoplastic rubber folding film (5) is adhered to the guide wire bag (2); and wherein the protective bag (4) is glued and connected with an adhesive tape (6); wherein a front surface of the guide wire bag (2) opposite to the back surface of the guide wire bag (2) is provided with a disinfection connection port (10); and a second side of the guide wire bag (2) opposite to the first side of the guide wire bag (2) is sealed and connected with a drain plug (7).

2. The drape including the integrated guide wire use device according to claim 1, wherein the drape body (1) comprises a surface layer (11), a water-absorbing layer (12), an antibacterial layer (13) and a bottom lining layer (14), wherein a bottom of the surface layer (11) is stitched and connected to a top of the water-absorbing layer (12).

3. The drape including the integrated guide wire use device according to claim 2, wherein a bottom of the water-absorbing layer (12) is stitched and connected to a top of the antibacterial layer (13), and a bottom of the antibacterial layer (13) is stitched and connected to a top of the bottom lining layer (14).

4. The drape including the integrated guide wire use device according to claim 2, wherein the water-absorbing layer (12) is woven by blending polyacrylonitrile fiber and polyethylene oxide fiber, the antibacterial layer (13) has a material of bamboo charcoal fiber, and the bottom lining layer (14) has a material of pure cotton.

5. The drape including the integrated guide wire use device according to claim 1, wherein the drape body (1) is stitched and connected with a fixing line (9).

6. The drape including the integrated guide wire use device according to claim 1, wherein the guide wire bag (2) has a material of polyethylene (PE).

7. The drape including the integrated guide wire use device according to claim 2, wherein the surface layer (11) comprises a first surface layer (111), a second surface layer (112) and a third surface layer (113), wherein the first surface layer (111) is separately stitched and connected to surfaces of the second surface layer (112) and the third surface layer (113), and the second surface layer (112) is stitched and connected to the surface of the third surface layer (113).

8. The drape including the integrated guide wire use device according to claim 7, wherein the first surface layer (111) has a material of cobes21G PP+70G airlaid, wherein cobes21G PP+70G airlaid is a composite layer compounded by polypropylene (PP) with a gram weight of 21 grams per square meter and airlaid with a gram weight of 70 grams per square meter, the second surface layer (112) has a material of transparent polyethylene (PE) with a thickness of 0.05 mm, and the third surface layer (113) has a material of cobes2730/pe27a+spu30B, wherein cobes2730/pe27a+spu30B is a composite layer compounded by polyethylene (PE) with a gram weight of 27 grams per square meter and blue polypropylene (PP) with a gram weight of 30 grams per square meter.

9. The drape including the integrated guide wire use device according to claim 1, wherein an outer side of the protective bag (4) is fixedly provided with a convex block (15), and wherein a material of the convex block (15) is polyethylene.

10. The drape including the integrated guide wire use device according to claim 1, wherein an end part of the second side of the guide wire bag (2) is a horn-shaped connector that expands toward the end of the second side of the guide wire bag (2), and the disinfection connection port (10) and the drain plug (7) are provided on the horn-shaped connector, and a protective film is encapsulated at a port of the disinfection connection port (10).

## Patentansprüche

1. Abdeckungstuch mit einer integrierten Führungsdraht-Verwendungsvorrichtung, das einen Tuchkörper (1) und einen Führungsdrahtsack (2) umfasst, wobei eine Rückfläche des Führungsdrahtsacks (2) beweglich mit dem Tuchkörper (1) verbunden ist, wobei die Rückfläche des Führungsdrahtsacks (2) mit einem Klebegewebe (8) verklebt und verbunden ist und das Klebegewebe (8) an den Tuchkörper (1) geklebt ist, und wobei ein innerer Hohlraum des Führungsdrahtsacks (2) fest mit einem Führungsdrahteinführungsblock (3) verbunden ist; wobei der Führungsdrahteinführungsblock (3) ein Material aus Schwamm oder weichem gummiertem Gewebe aufweist und eine erste Seite des Führungsdrahtsacks (2) beweglich mit einem Schutzsack (4) verbunden ist; wobei der Schutzsack (4) mit einer thermoplastischen Gummifaltfolie (5) verklebt ist und die thermoplastische Gummifaltfolie (5) an den Führungsdrahtsack (2) geklebt ist; und wobei der Schutzsack (4) mit einem Klebeband (6) verklebt und verbunden ist; wobei eine vordere Fläche des Führungsdrahtsacks (2), die der hinteren Fläche des Führungsdrahtsacks (2) gegenüberliegt, mit einer Desinfektionsanschlussöffnung (10) versehen ist, und eine zweite Seite des Führungsdrahtsacks (2), die der ersten Seite des Führungsdrahtsacks (2) gegenüberliegt, mit einem Ablaufstopfen (7) versiegelt und verbunden ist.

2. Abdeckungstuch mit integrierter Führungsdraht-Verwendungsvorrichtung nach Anspruch 1, wobei der Tuchkörper (1) eine Oberflächenschicht (11), eine wasserabsorbierende Schicht (12), eine antibakterielle Schicht (13) und eine Bodenauskleidungsschicht (14) umfasst, wobei eine Unterseite der Oberflächenschicht (11) mit einer Oberseite der wasserabsorbierenden Schicht (12) vernäht und verbunden ist.

3. Abdeckungstuch mit integrierter Führungsdraht-Verwendungsvorrichtung nach Anspruch 2, wobei eine Unterseite der wasserabsorbierenden Schicht (12) mit einer Oberseite der antibakteriellen Schicht (13) vernäht und verbunden ist, und eine Unterseite der antibakteriellen Schicht (13) mit einer Oberseite der Bodenauskleidungsschicht (14) vernäht und verbunden ist.

4. Abdeckungstuch mit integrierter Führungsdraht-Verwendungsvorrichtung nach Anspruch 2, wobei die wasserabsorbierende Schicht (12) durch Mischen von Polyacrylnitrilfasern und Polyethylenoxidfasern gewebt ist, die antibakterielle Schicht (13) ein Material aus Bambuskohlefasern aufweist und die Bodenauskleidungsschicht (14) ein Material aus reiner Baumwolle aufweist.

5. Abdeckungstuch mit integrierter Führungsdraht-Verwendungsvorrichtung nach Anspruch 1, wobei der Tuchkörper (1) mit einer Befestigungslinie (9) vernäht und verbunden ist.

6. Abdeckungstuch mit integrierter Führungsdraht-Verwendungsvorrichtung nach Anspruch 1, wobei der Führungsdrahtsack (2) ein Material aus Polyethylen (PE) aufweist.

7. Abdeckungstuch mit integrierter Führungsdraht-Verwendungsvorrichtung nach Anspruch 2, wobei die Oberflächenschicht (11) eine erste Oberflächenschicht (111), eine zweite Oberflächenschicht (112) und eine dritte Oberflächenschicht (113) umfasst, wobei die erste Oberflächenschicht (111) mit Oberflächen der zweiten Oberflächenschicht (112) und der dritten Oberflächenschicht (113) separat genäht und verbunden ist, und die zweite Oberflächenschicht (112) mit der Oberfläche der dritten Oberflächenschicht (113) genäht und verbunden ist.

8. Abdeckungstuch mit der integrierten Führungsdraht-Verwendungsvorrichtung nach Anspruch 7, wobei die erste Oberflächenschicht (111) ein Material aus cobes21G PP+70G airlaid aufweist, wobei cobes21G PP+70G airlaid eine Verbundschicht ist, die aus Polypropylen (PP) mit einem Grammgewicht von 21 Gramm pro Quadratmeter und airlaid mit einem Grammgewicht von 70 Gramm pro Quadratmeter zusammengesetzt ist, wobei die zweite Oberflächenschicht (112) ein Material aus transparentem Polyethylen (PE) mit einer Dicke von 0. 05 mm aufweist, und wobei die dritte Oberflächenschicht (113) ein Material aus cobes2730/pe27a+spu30B aufweist, wobei cobes2730/pe27a+spu30B eine Verbundschicht ist, die aus Polyethylen (PE) mit einem Grammgewicht von 27 Gramm pro Quadratmeter und blauem Polypropylen (PP) mit einem Grammgewicht von 30 Gramm pro Quadratmeter zusammengesetzt ist.

9. Abdeckungstuch mit integrierter Führungsdraht-Verwendungsvorrichtung nach Anspruch 1, wobei eine Außenseite des Schutzsacks (4) fest mit einem konvexen Block (15) versehen ist, und wobei ein Material des konvexen Blocks (15) Polyethylen ist.

10. Abdeckungstuch mit integrierter Führungsdraht-Verwendungsvorrichtung nach Anspruch 1, wobei ein Endteil der zweiten Seite des Führungsdrahtsacks (2) ein hornförmiges Verbindungsstück ist, das sich zum Ende der zweiten Seite des Führungsdrahtsacks (2) hin erweitert, und wobei die Desinfektionsanschlussöffnung (10) und der Ablaufstopfen (7) an dem hornförmigen Verbindungsstück vorgesehen sind, und wobei eine Schutzfolie an einer Öffnung der Desinfektionsanschlussöffnung (10) eingekapselt ist.

## Revendications

1. Un drap comprenant un dispositif intégré d'utilisation du fil-guide, comprenant un corps de drap (1) et un sac de fil-guide (2), dans lequel une surface arrière du sac de fil-guide (2) est reliée de manière mobile au corps du drap (1), dans lequel la surface arrière du sac de fil guide (2) est collée et reliée à un tissu adhésif (8) ; et le tissu adhésif (8) est collé au corps de drapé (1), et dans lequel une cavité intérieure du sac de fil guide (2) est reliée de manière fixe à un bloc d'insertion de fil guide (3) ; dans lequel le bloc d'insertion de fil guide (3) a un matériau en éponge ou en tissu caoutchouté souple ; et un premier côté du sac de fil guide (2) est relié de manière mobile à un sac de protection (4) ; dans lequel le sac de protection (4) est adhéré à un film pliable en caoutchouc thermoplastique (5), et le film pliable en caoutchouc thermoplastique (5) est adhéré au sac de fil-guide (2) ; et dans lequel le sac de protection (4) est collé et relié à une bande adhésive (6) ; dans lequel une surface avant du sac de fil de guidage (2) opposée à la surface arrière du sac de fil de guidage (2) est pourvue d'un port de connexion de désinfection (10) ; et un deuxième côté du sac de fil de guidage (2) opposé au premier côté du sac de fil de guidage (2) est scellé et connecté à un bouchon de vidange (7).

2. Le drap comprenant le dispositif intégré d'utilisation du fil-guide selon la revendication 1, dans lequel le corps du drap (1) comprend une couche de surface (11), une couche absorbant l'eau (12), une couche antibactérienne (13) et une couche de doublure inférieure (14), dans laquelle une partie inférieure de la couche de surface (11) est cousue et reliée à une partie supérieure de la couche absorbant l'eau (12).

3. Le drap comprenant le dispositif intégré d'utilisation du fil-guide selon la revendication 2, dans lequel une partie inférieure de la couche absorbant l'eau (12) est cousue et reliée à une partie supérieure de la couche antibactérienne (13), et une partie inférieure de la couche antibactérienne (13) est cousue et reliée à une partie supérieure de la couche de doublure inférieure (14).

4. Le drap comprenant le dispositif intégré d'utilisation du fil-guide selon la revendication 2, dans lequel la couche d'absorption d'eau (12) est tissée en mélangeant des fibres de poly acrylonitrile et des fibres d'oxyde de polyéthylène, la couche antibactérienne (13) est composée de fibres de charbon de bambou, et la couche de doublure inférieure (14) est composée de coton pur.

5. Le drap comprenant le dispositif intégré d'utilisation du fil-guide selon la revendication 1, dans lequel le corps du drap (1) est cousu et relié à une ligne de fixation (9).

6. Le drap comprenant le dispositif intégré d'utilisation du fil-guide selon la revendication 1, dans lequel le sac du fil-guide (2) a un matériau en polyéthylène (PE).

7. Le drap comprenant le dispositif intégré d'utilisation du fil-guide selon la revendication 2, dans lequel la couche de surface (11) comprend une première couche de surface (111), une deuxième couche de surface (112) et une troisième couche de surface (113), dans laquelle la première couche de surface (111) est cousue séparément et reliée aux surfaces de la deuxième couche de surface (112) et de la troisième couche de surface (113), et la deuxième couche de surface (112) est cousue et reliée à la surface de la troisième couche de surface (113).

8. Le drap comprenant le dispositif intégré d'utilisation du fil-guide selon la revendication 7, dans lequel la première couche de surface (111) a un matériau de cobes21G PP+70G airlaid, dans lequel cobes21G PP+70G airlaid est une couche composite composée de polypropylène (PP) avec un poids de 21 grammes par mètre carré et airlaid avec un poids de 70 grammes par mètre carré, la deuxième couche de surface (112) a un matériau de polyéthylène (PE) transparent d'une épaisseur de 0. 05 mm, et la troisième couche de surface (113) est en cobes2730/pe27a+spu30B, où cobes2730/pe27a+spu30B est une couche composite composée de polyéthylène (PE) d'un poids de 27 grammes par mètre carré et de polypropylène bleu (PP) d'un poids de 30 grammes par mètre carré.

9. Le drap comprenant le dispositif intégré d'utilisation du fil-guide selon la revendication 1, dans lequel un côté extérieur du sac de protection (4) est pourvu de façon fixe d'un bloc convexe (15), et dans lequel un matériau du bloc convexe (15) est le polyéthylène.

10. Le drap comprenant le dispositif intégré d'utilisation du fil-guide selon la revendication 1, dans lequel une partie de l'extrémité du deuxième côté du sac de fil-guide (2) est un connecteur en forme de corne qui s'étend vers l'extrémité du deuxième côté du sac de fil-guide (2), et le port de connexion de désinfection (10) et le bouchon de vidange (7) sont fournis sur le connecteur en forme de corne, et un film protecteur est encapsulé dans un port du port de connexion de désinfection (10).
